# EUROPÄISCHE PATENTANMELDUNG

(11) **EP 4 091 651 A1**
(43) Veröffentlichungstag der Anmeldung: **23.11.2022**
(21) Anmeldenummer: 22174039.2
(22) Anmeldetag: 18.05.2022
(51) Int. Cl.: A61M 5/315

(54) **BI-DOSE-VORRICHTUNG**

(30) Priorität: 19.05.2021 DE 102021112962
(71) Anmelder: F+K Innovationen GmbH & Co.KG, 76534 Baden-Baden (DE)
(72) Erfinder: FUCHS, Karl-Heinz, 78315 Radolfzell (DE); KELLER, Alexander, 82401 Rottenburg (DE); HUTTER, Ludwig, 82497 Unterammergau (DE)
(74) Vertreter: Patentanwälte und Rechtsanwalt Weiß, Arat & Partner mbB

(57) **Zusammenfassung**

Die Erfindung betrifft eine BI-Dose-Vorrichtung (1) bestehend aus einem Flüssigkeitskartusche (2) und einer Steuerung (3), wobei die Flüssigkleitskartusche (2) mittels einer Betätigungskolbens (6) entleerbar ist, wobei der Betätigungskolben (6) eine erste Dosierklammer (7) und eine zweite Dosierklammer (8) aufweist, wobei die beiden Dosierklammern (7, 8) miteinander in Wirkverbindung stehen.

## Beschreibung

### Technisches Gebiet

Die Erfindung betrifft eine BI-Dose-Vorrichtung nach dem Oberbegriff des Anspruchs 1.

### Stand der Technik

BI-Dose Systeme sind allgemein bekannt und werden in der Pharma als vielfältige Einmalanwendungen für Applikationsmengen im Bereich von 50 bis 200 Mikroliter oral, vaginal oder nasal eingesetzt.

Um die beiden gewünschten Dosiermengen unabhängig voneinander aus dem System zu applizieren, bedarf es einer Dosiersteuerung.

Bekannte BI-Dose Dosiersysteme regeln die Ausbringmengen über ein Stufenkolbenprinzip ohne zusätzlichen Dosierschutz.

Vor unbeabsichtigtem Betätigen und vor manipulativen Eingriffen sind die Systeme nicht ausreichend geschützt und gesichert.

In diesem Zusammenhang wird auf die EP 2 773 406 hingewiesen, welche ein Dosiersystem mit auswechselbaren Einmalkartuschen für beide oder mehrere Applikationen beinhaltet.

### Aufgabe der Erfindung

Die Aufgabe der vorliegenden Erfindung ist es, die Nachteile aus dem Stand der Technik zu überwinden und die neue Idee soll auch für große Ausbringmengen pro Betätigungshub einsetzbar sein.

### Lösung der Aufgabe

Zur Lösung der Aufgabe führen die Merkmale nach dem Anspruch 1. Vorteilhafte Ausgestaltungen sind in den Unteransprüchen beschrieben.

Die neue Idee ist für kleine Applikationsmengen bis zu 200 Mikroliter, aber auch für große Applikationsmengen bis zu 2 Milliliter und größer pro Betätigungshub einsetzbar.

Dabei kommt eine erfindungsgemäße BI-Dose-Vorrichtung zum Einsatz. Die BI-Dose-Vorrichtung wird im Rahmen der Erfindung auch als BI-Dose-System bezeichnet.

Das teilereduzierte System besteht aus zwei Baugruppen der Mediumkartusche mit verschiebbarem Betätigungs- und Verschlussstopfen sowie der Baueinheit Verdrängungskolben mit den Dosiervolumensteuerungs- und Verankerungsklammern mit integriertem Dosierschutz sowie den integrierten BI-Dose Funktionen und den selbsterklärenden Anwendungsfunktionen.

Der Systemaufbau besteht aus einer Kartusche für das auszubringende Medium. Im vorderen Kartuschenbereich ist eine Produktauslassöffnung als Spray- oder Strahlausbringung angeordnet.

Und im unteren Kartuschenbereich ist ein beweglicher, verschiebbarer Stopfen als Mediumverschluss integriert, welcher über einen manuell zu betätigenden Kolben gegen das gelagerte Medium verschoben wird und dadurch die Flüssigkeit über die Kartuschenauslassöffnung ausbringt.

Über die am unteren Ende der Kartusche fixierten Fingerauflagefläche und dem am Kolben beweglichen Kolbenflansch wird das System manuell betätigt.

Die am Kolben zwischen Fingerauflage der Kartusche und beweglichem Kolbenflansch abnehmbaren eingerasteten Abziehklammern steuern den Kolbenhub und somit das eingestellte Ausbringvolumen des BI-Dose Systems.

Die Verankerung beider Baugruppen Kartusche und Verdrängerkolben wird über die aufgesteckte erste Abziehklammer erreicht, indem die Klammer mit einem integrierten Aufnahmeschlitz sich über und unter dem Fingerauflageflansch schiebt, sich fixiert und somit beide Baugruppen in der Ruheposition auch während des Transports miteinander verbindet.

Durch das Abziehen der ersten Klammer werden die beiden Baugruppen für den Applikationseinsatz manuell zueinander verschiebbar getrennt.

Über die Skalierung auf der glasklaren Kartuschenoberfläche kann der Anwender visuell das Ausbringen des Mediums verfolgen und hat somit visuell die Kontrolle über die einzelnen Dosierhübe.

Nach dem Abziehen der Systemschutz- und Dichtkappe wird anschließend vom Anwender ebenfalls die erste Dosierklammer abgezogen, um den ersten Hub über den Kolben betätigen zu können. Für den zweiten Hub zieht der Benutzer die zweite Dosierklammer ebenfalls vom Kolben ab und betätigt erneut den Kolben über den Flansch.

Die erste Dosierklammer blockiert über den Fingerflansch der Kartusche eine unbeabsichtigte Betätigung des Systems und ist auch als Transportsicherung sowie für die Verankerung beider Baugruppen ausgelegt.

Gegenüber der Blockier- und Sicherungssegmente im Fingerauflagenbereich überlappt die erste Dosierklammer die zweite Dosierklammer im Bereich des Außendurchmessers sowie über den kompletten Abzugsgriff der zweiten Klammer, so dass die zweite Klammer nicht vor der ersten Klammer abgezogen werden kann.

Mit der Überlappung der zweiten Klammer durch die erste Klammer wird dem Anwender auch der Handlings-, Dosier-, und Applikationsablauf durch das System selbsterklärend aufgezeigt.

Umlaufende Nuten am Betätigungskolbenaußendurchmesser legen die Dosierhublänge und somit das Ausbringvolumen fest.

Je nach Position der Nuten können verschiedene Dosiervolumen aus dem System ausgebracht werden.

In die umlaufenden Nuten werden die Abziehklammern eingerastet und blockieren im eingerasteten Zustand die Betätigung des Systems.

Nach dem Abziehen der ersten Klammer kann der Betätigungskolben bis zum Plananschlag der zweiten Klammer an der unteren Fläche der Fingerauflage bzw. Kartuschenwand betätigt werden und somit wird der Stopfen in der Kartusche gegen die Flüssigkeit verschoben, verdrängt sie, um die erste dosierte Applikation zu erhalten.

Mit dem anschließenden Abziehen der zweiten Klammer kann der Kolben bis zum Anschlag des Stopfens im vorderen Bereich der Kartusche betätigt werden und bringt somit die zweite Dosierung zur Applikation.

Eine angeordnete Verdrehsicherung zwischen den beiden Abziehklammern blockiert die Torsion der beiden Elemente gegeneinander, so dass es nicht möglich ist, die zweite Klammer unter der ersten Klammer durchzudrehen und das System zu manipulieren.

Beide Baugruppen sind auch autark einsetzbar.

Die Kartuschenbaugruppe kann als funktionstüchtige Einheit auch als Einmalanwendung mit einem separaten Betätigungskolben oder in anderen Applikationsgeräten zum Einsatz kommen.

Die BI-Dose Steuerung als Baugruppe mit dem Kolben und den zwei oder mehreren Abziehklammern kann als Betätigungsschutz, Produktschutz, Dosierschutz, Dosiersteuerung als Aufrüsteinheit verschiedenster Applikationsgeräte vervollständigen.

Die neue Dosiersteuerung garantiert eine saubere, genau dosierte Applikation auch bei mehreren Dosiervolumen und bei unterschiedlichen Volumenmengen bei bekanntem Handling.

Das teilereduzierte System ist für eine automatische Montage sowie das automatische Abfüllen der Kartuschen konstruiert.

### Figurenbeschreibung

Weitere Vorteile, Merkmale und Einzelheiten der Erfindung ergeben sich aus der nachfolgenden Beschreibung bevorzugter Ausführungsbeispiele sowie anhand der Zeichnungen; diese zeigen in:
- Figur 1: eine 3D-Ansicht des erfindungsgemäßen BI-Dose Systems mit Dosierschutz;
- Figur 2: einen Längsschnitt des erfindungsgemäßen BI- Dose Systems mit Dosierschutz;
- Figur 3: einen Schnitt durch die autarke Baugruppe 2 der Kartusche des erfindungsgemäßen BI-Dose Systems;
- Figur 4: eine Teil 3D-Ansicht der autarken Baugruppe 3 der Steuerung des erfindungsgemäßen BI-Dose Systems;
- Figur 5: einen Teilschnitt der Baugruppe 3, die Steuerung des erfindungsgemäßen BI-Dose Systems;
- Figur 6: zeigt einen Teilschnitt der Baugruppe 3, die Verdrehsicherung der Steuerung des erfindungsgemäßen BI-Dose Systems.

### Ausführungsbeispiel

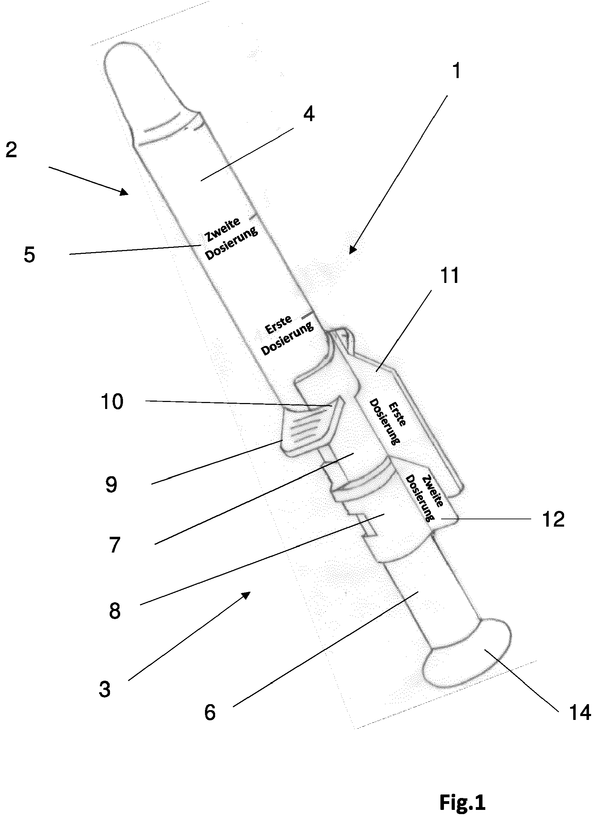

Die Figur 1 zeigt eine 3D-Ansicht der erfindungsgemäßen BI-Dose mit Dosierschutz 1, bestehend aus den autarken Baugruppen Flüssigkeitskartusche 2 und der autarken Baugruppe BI-Dose Steuerung 3. Die Baugruppe 2 mit dem Kartuschenkörper 4 der Dosiervolumen-Skalierung 5 auf der Kartuschenkörperoberfläche.

Die Baugruppe 3 besteht aus dem Betätigungskolben 6 und den beiden Abziehklammern, der ersten Dosierung 7 sowie der zweiten Dosierung 8.

Die beiden Baugruppen werden über den Außendurchmesser und der Fingerauflage 9, angeordnet an der Kartuschenwand, verbunden.

Die Baugruppe 3 fixiert und zentriert sich über den Kolbenaußendurchmesser 6 im Kartuscheninnendurchmesser und verankert beide Baugruppen über die erste Dosierklammer 7 durch einen Einschnitt 10 mit der Fingerauflage 9.

Die Dosierklammern 7 und 8 für die erste und zweite Dosierung umklammern den Kolben- und den Kartuschenaußendurchmesser ca. zwei Drittel des Umfanges, so dass trotz einer sicheren Klammerfixierung auf dem Kolben und Verankerung an der Kartusche über die Fingerauflage 9 auch ein sicheres Abziehen der Klammeren über die Griffe 11 und 12 möglich ist.

In der dargestellten Ruheposition verriegelt die erste Dosierklammer 7 das komplette BI-Dose Dosierschutzsystem 1.

Über die Verdrehsicherung 13 und den überlappenden Abziehgriffen 11 und 12 wird garantiert, dass die Applikationsfunktionen selbsterklärend abfolgen.

Im ersten Schritt muss die erste Dosierklammer 7 über den Griff 11 vom Benutzer entfernt werden, um das System zu entsperren und um über den Betätigungskolben 6 die erste Applikation freizugeben.

Nach dem Abziehen der zweiten Dosierklammer 8 über den Griff 12 kann mit dem Kolben 6 über den Kolbenflansch 14 die zweite Applikation durchgeführt werden.

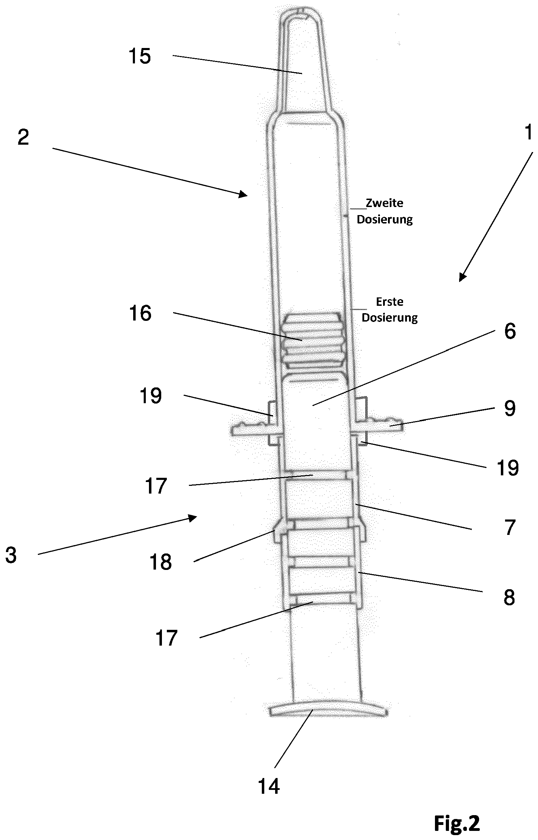

Die Figur 2 zeigt einen Längsschnitt durch die erfindungsgemäße BI-Dose mit Dosierschutz 1 und den beiden autarken Baugruppen 2 und 3.

Der Kartuschenkörper der Baugruppe 2 beinhaltet für die Spray- oder Strahlfunktion einen Amboss 15 und gegenüber des Amboss im hinteren Kartuschenbereich den beweglich verschiebbaren Verschlussstopfen 16.

Der Betätigungskolben 6 mit dem Kolbenflansch 14 sitzt hinter dem Verschlussstopfen 16 und zentriert die Baugruppe 3 in der Baugruppe 2. In die am Kolbendurchmesser umlaufenden Dosiernuten 17 rasten die Abziehklammern 7 und 8 ein und geben dem System die Hublängen für die gewünschten Dosiervolumen vor.

Durch die Durchmesserüberlappung 18 der ersten Dosierklammer 7 über die zweite Dosierklammer 8 ist der Handlingsablauf gesichert und das Abziehen der ersten Klammer vorgegeben, wobei die erste Klammer 7 mit dem Einschub 19 in die Fingerauflage 9 greift und somit die Baugruppen in der Ruheposition zusammenhält, um nach dem Abziehen der ersten Klammer 7 die erste Dosierung zur Betätigung über den Kolben 6 frei zu geben.

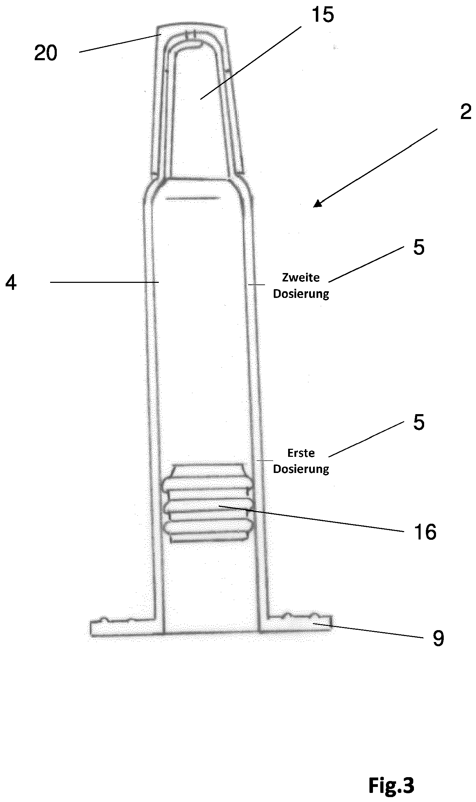

Die Figur 3 zeigt einen Längsschnitt durch die autarke Baugruppe 2 mit dem Flüssigkeitskartuschenkörper 4 mit aufgesetzter Schutz- und Dichtkappe 20, die am Umfang integrierte Fingerauflage 9 sowie den nach der Befüllung gesetzten Verschlussstopfen 16.

Die am Außendurchmesser der Flüssigkeitskartusche 4 markierte Dosiervolumenskalierung 5 zeigt dem Anwender die beiden zu verabreichenden dosierten Ausbringvolumen an.

Über den im vorderen Bereich integrierten Amboss 15 wird ein Spray oder Strahl je nach Wunsch erzeugt.

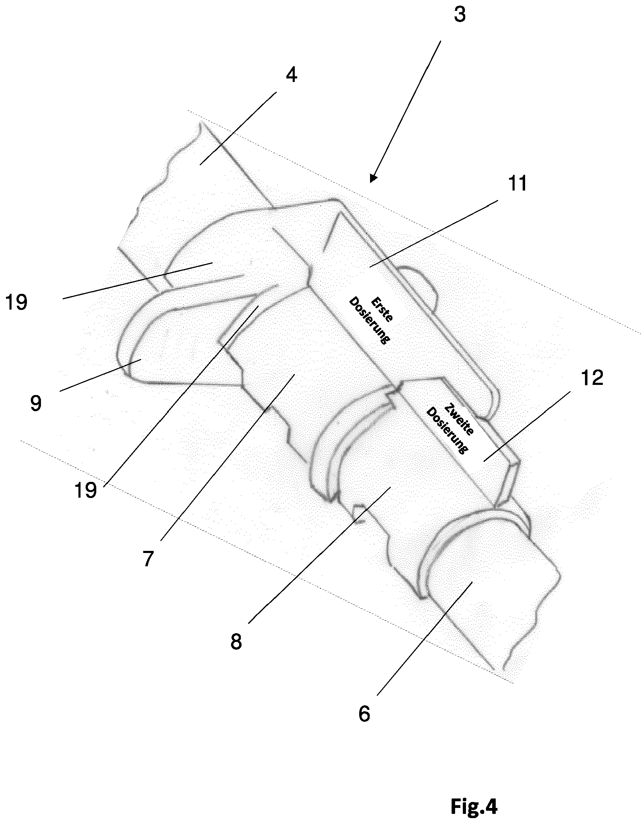

Die Figur 4 zeigt eine Teil-3D-Ansicht der autarken Baugruppe 3 mit dem Betätigungskolben 6, dem Kartuschenkörper 4 mit der Fingerauflage 9 der Baugruppe 2 sowie den beiden Abziehklammern 7 und 8 mit den Griffen 11 und 12 das Herz der Baugruppe 3.

Über den Einschub 19 werden beide Baugruppen 2 und 3 durch den Fingerflansch 9 fixiert und verankert.

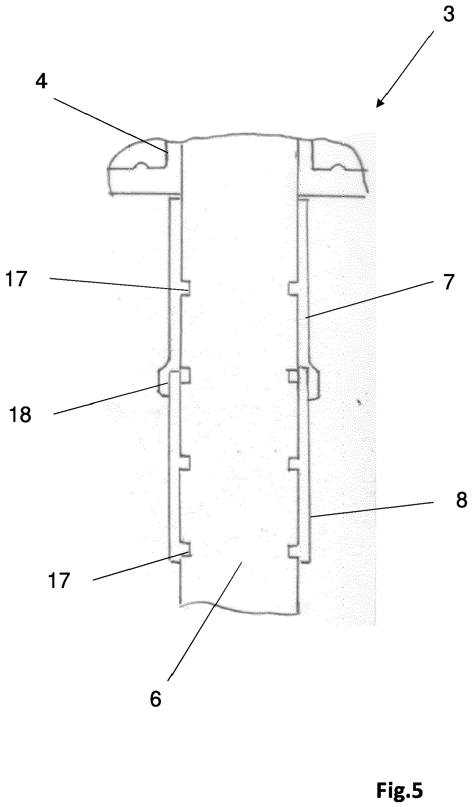

Die Figur 5 zeigt einen Teilschnitt durch die Baugruppen 3 mit der angedeuteten Kartusche 4 aus der Baugruppe 2.

Die am Kolben 6 umlaufenden Nuten 17 nehmen die beiden Abziehklammern 7 und 8 auf.

Die Überlappung 18 fixiert beide Klammern 7 und 8 auf dem Kolben 6, in den Nuten 17 eingerastet, geben sie den Dosierweg des Systems vor.

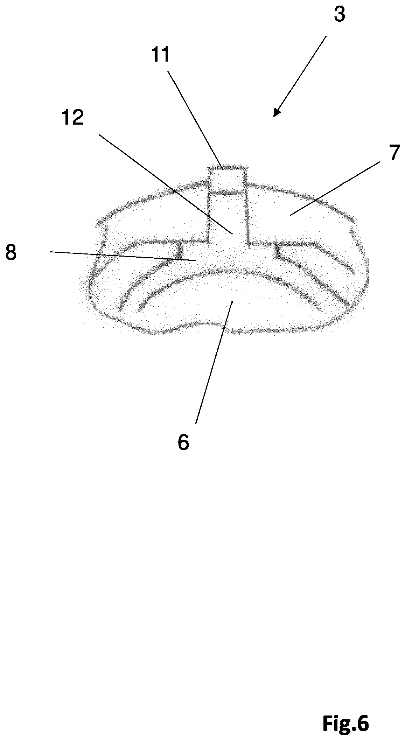

Die Figur 6 zeigt einen Teilschnitt der autarken Baugruppe 3 mit der Verdrehsicherung 13 zur Sicherung der beiden Klammern 7 und 8 auf dem Kolben 6.

Durch eine Aussparung am Überlappungsdurchmesser der ersten Dosierklammer 7 im Bereich des Griffes 11 greift der Griff 12 beim Aufsetzen der Klammer 7 in die Aussparung und bildet dadurch die Verdrehsicherung 13.

**Bezugszeichenliste**

| | |
|---|---|
| 1 | BI-Dose-Vorrichtung |
| 2 | Baugruppe Flüssigkeitskartusche |
| 3 | Baugruppe BI-Dose Steuerung |
| 4 | Kartuschenkörper |
| 5 | Dosiervolumenskalierung |
| 6 | Betätigungskolben |
| 7 | erste Dosierklammer |
| 8 | zweite Dosierklammer |
| 9 | Fingerauflage |
| 10 | Einschnitt |
| 11 | erster Dosierklammer Griff |
| 12 | zweiter Dosierklammer Griff |
| 13 | Verdrehsicherung |
| 14 | Kolbenflansch |
| 15 | Amboss |
| 16 | Verschlussstopfen |
| 17 | Nuten |
| 18 | Überlappung |
| 19 | Einschub |
| 20 | Schutz- und Dichtkappe |
| 21 | |
| 22 | |
| 23 | |

## Patentansprüche

1. BI-Dose-Vorrichtung (1) bestehend aus einer Flüssigkeitskartusche (2) und einer Steuerung (3), wobei die Flüssigkleitskartusche (2) mittels eines Betätigungskolbens (6) entleerbar ist,
**dadurch gekennzeichnet, dass**
der Betätigungskolben (6) eine erste Dosierklammer (7) und eine zweite Dosierklammer (8) aufweist, wobei die beiden Dosierklammern (7, 8) verdrehgesichert miteinander in Wirkverbindung stehen.

2. BI-Dose-Vorrichtung nach Anspruch 1, **dadurch gekennzeichnet, dass** die beiden Dosierklammern (7, 8) auf einer Betätigungskolbenkörperoberfläche entfernbar aufgeclipst sind.

3. BI-Dose-Vorrichtung nach einem der vorigen Ansprüche, **dadurch gekennzeichnet, dass** die erste Dosierklammer (7) für eine erste Dosierung den Kolben- und den Kartuschenaußendurchmesser teilweise umklammert und eine Klammerfixierung auf dem Betätigungskolben (6) und Verankerung an der Kartusche (4) aufweist.

4. BI-Dose-Vorrichtung nach einem der vorigen Ansprüche, **dadurch gekennzeichnet, dass** die zweite Dosierklamer (8) für eine zweite Dosierung den Kolbenaußendurchmesser teilweise umklammert und eine Klammerfixierung auf dem Betätigungskolben (6) aufweist.

5. BI-Dose-Vorrichtung nach einem der vorigen Ansprüche, **dadurch gekennzeichnet, dass** die Steuerung (3) sich über den Kolbenaußendurchmesser des Betätigungskolbens (6) im Innendurchmesser der Flüssigkeitskartusche (2) verankert und die Flüssigkeitskartusche (2) und die Steuerung (3) über die erste Dosierklammer (7) durch einen Einschnitt (10) mit der Fingerauflage (9) verbunden ist.

6. BI-Dose-Vorrichtung nach einem der vorigen Ansprüche, **dadurch gekennzeichnet, dass** die beiden Dosierklammern (7, 8) In die am Kolbendurchmesser umlaufenden Dosiernuten (17) einrasten und geben die Hublängen für die gewünschten Dosiervolumen vor.

7. BI-Dose-Vorrichtung nach einem der vorigen Ansprüche, **dadurch gekennzeichnet, dass** eine Durchmesserüberlappung (18) der ersten Dosierklammer (7) über die zweite Dosierklammer (8) lappt, sodass das Abziehen zunächst der ersten Dosierklammer (7) vorgegeben ist, wobei die erste Dosierklammer (7) mit einem Einschub (19) in eine Fingerauflage (9) greift und somit die Baugruppen in der Ruheposition zusammenhält, wobei nach dem Abziehen der ersten Dosierklammer (7) eine erste Dosierung zur Betätigung über den Kolben 6 freigibt.

8. BI-Dose-Vorrichtung nach einem der vorigen Ansprüche, **dadurch gekennzeichnet, dass** eine Verdrehsicherung zwischen den beiden Dosierklammern (7, 8 ) über die Griffe (11,12) vorgegeben ist.
